(19) Europäisches Patentamt
European Patent Office
Office européen des brevets

(11) Publication number: 0 117 053
A1

# (12) EUROPEAN PATENT APPLICATION

(21) Application number: 84300256.9

(22) Date of filing: 17.01.84

(51) Int. Cl.³: C 07 D 205/08
C 07 D 403/12, C 07 D 417/12
//A61K31/395

(30) Priority: 10.02.83 JP 21285/83
15.12.83 JP 236953/83

(43) Date of publication of application:
29.08.84 Bulletin 84/35

(84) Designated Contracting States:
CH DE FR GB IT LI NL SE

(71) Applicant: AJINOMOTO CO., INC.
5-8, Kyobashi 1-chome, Chuo-ku
Tokyo 104(JP)

(72) Inventor: Kato, Toshihisa
No. 2-20-8, Kannon Kawasaki-ku
Kawasaki-shi Kanagawa-ken(JP)

(72) Inventor: Nakanishi, Eiji
No. 2-20-8, Kannon Kawasaki-ku
Kawasaki-shi Kanagawa-ken(JP)

(72) Inventor: Yasuda, Naohiko
No. 2-36-2, Hairando
Yokosuka-shi Kanagawa-ken(JP)

(72) Inventor: Sasaki, Yukio
No. 2-20-8, Kannon Kawasaki-ku
Kawasaki-shi Kanagawa-ken(JP)

(74) Representative: Bond, Bentley George et al,
Haseltine Lake & Co. 28 Southampton Buildings
Chancery Lane
London WC2A 1AT(GB)

## (54) Azetidinone derivatives.

(57) There are disclosed azetidinone derivatives of the general formula:

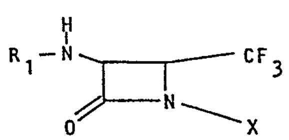

wherein $R_1$ is an acyl group, a hydrogen atom or an amino-protecting group, and X is a hydrogen atom, an alkyloxy group, an aralkyloxy group, an aryloxy group, the group $-OH$, the group $-OSO_3^-M^+$ wherein $M^+$ is a cation, or the group $-SO_3^-M^+$ wherein $M^+$ is a cation. Derivatives wherein $R_1$ is an acyl group have antibacterial activity, whilst derivatives wherein $R_1$ is a hydrogen atom or an amino-protecting group are useful as intermediates in the production of derivatives wherein $R_1$ is an acyl group.

EP 0 117 053 A1

## AZETIDINONE DERIVATIVES

The present invention relates to azetidinone derivatives useful as antibiotics and as intermediates for the production of such antibiotics.

The present invention provides 4-trifluoromethylazetidinone derivatives (including salts thereof) of the general formula:

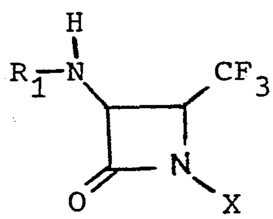

(I)

wherein $R_1$ is an acyl group, a hydrogen atom or an amino-protecting group, and X is a hydrogen atom, an alkyloxy group, an aralkyloxy group, an aryloxy group, the group -OH, the group $-OSO_3^-M^+$ wherein $M^+$ is a cation, or the group $-SO_3M^+$ wherein $M^+$ is a cation. Examples of the cation $M^+$ are

$H^+$, 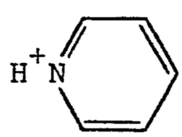, $n\text{-}Bu_4N^+$ and $Na^+$.

The derivatives are novel, and are useful as highly active β-lactam antibiotics or as intermediates. Thus, the derivatives wherein $R_1$ is an acyl group have been found to have a wide antibacterial spectrum against Gram negative bacteria, including *Pseudomonas aeruginosa*, and Gram positive bacteria, and are

useful as antibiotics, while the derivatives wherein $R_1$ is a hydrogen atom or an amino-protecting group are useful as intermediates in the production of derivatives wherein $R_1$ is an acyl group.

Examples of the acyl group $R_1$ include acyl groups, substituted at the 6-position, of conventional penicillin derivatives, and acyl groups, substituted at the 7-position, of cephalosporin derivatives, for example, the following acyl groups:

(1) Acyl groups of the formula:

$$R_2CO-$$

wherein $R_2$ is a lower alkyl group, an optionally-substituted phenyl group, a heterocyclic group, or an optionally-substituted benzoyl group.

(2) Acyl groups of the formula:

$$\begin{array}{c} R_3NHCHCO- \\ \quad\ \ | \\ \quad\ \ R_5 \end{array}$$

wherein $R_5$ is a hydrogen atom, an optionally-substituted amino acid residue, an amino-protecting group, a group of the formula $R_4-(CH_2)_{n'}-CO-$ [wherein $R_4$ is an optionally-substituted heterocyclic group, an optionally-substituted phenyl group, an optionally-substituted lower alkyl group, an optionally-substituted phenylthio group, or an optionally-substituted lower alkylthio group, n' is 0 or an integer of 1 to 4, and $-(CH_2)_{n'}-$ may optionally contain one or more substituents], a group of the formula

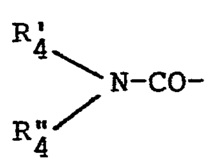

[wherein $R_4'$ and $R_4''$ are the same or different and each is a hydrogen atom, a lower alkyl group, a lower alkylcarbamoyl group, an optionally-substituted phenylcarbamoyl group, or an optionally-substituted sulphonic acid group, or $R_4'$ and $R''_4$ together are a cyclic hydrocarbon ring which may optionally contain one or more nitrogen atoms and/or

one or more oxygen atoms and/or one or more other hetero atoms and whose carbon atom(s) and/or nitrogen atom(s) may optionally contain substituents], or a group of the formula $R_4'''-SO_2-$ [wherein $R_4'''$ is an optionally-substituted lower alkyl group]; and $R_5$ is a hydrogen atom, an optionally-substituted lower alkyl group, an optionally-substituted phenyl group, an optionally-substituted heterocyclic group, a cycloalkenylene group, or a combination of a heterocyclic ring and carbonylamino group which heterocyclic ring and carbonylamino group may optionally be combined via an optionally-substituted alkylene group.

Specific examples of acyl groups of this type are (4-substituted-2,3-dioxy-1-piperazinecarboxamido)-arylacetyl groups of the formula:

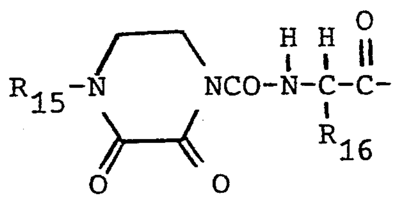

wherein $R_{16}$ is an aromatic group [e.g. a carbocyclic aromatic hydrocarbon group, e.g. of the formula

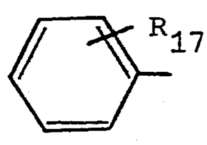

or the like, wherein $R_{17}$ is a hydroxyl group, a halogen atom, a cyano group, a nitro group, an amino group or a mercapto group], or a substituted or unsubstituted 5-, 6- or 7- membered heterocyclic ring containing 1, 2, 3 or 4 nitrogen and/or oxygen and/or sulphur atoms, and $R_{15}$ is an alkyl group, a substituted alkyl group [e.g. an alkyl group containing one or more halogen atoms, cyano groups, nitro groups, amino groups or mercapto groups] an arylmethyleneamino group [e.g. $-N=CHR_{16}'$ wherein

$R'_{16}$ has the same definition as for $R_{16}$ above], an arylcarbonylamino group [e.g.

$$-NH\overset{O}{\overset{\|}{C}}-R''_{16}$$

wherein $R''_{16}$ has the same definition as for $R_{16}$ above], or an alkylcarbonylamino group. Preferred examples thereof are those derivatives wherein $R_{15}$ is an ethyl group and $R_{16}$ is a phenyl group or a p-hydroxyphenyl group.

<u>(3) Acyl groups of the formula:</u>

$$R_6-R_7CO-$$

wherein $R_6$ is a group of the formula $R_8-\overset{|}{C}=N-X'-R_9$ [wherein X' is an oxygen or sulphur atom, $R_8$ is an optionally-substituted heterocyclic gorup or an optionally-substituted phenyl group, and $R_9$ is a hydrogen atom, an optionally-substituted phenyl group, an optionally-substituted lower acyl group, an optionally-substituted lower alkyl group, or a group of the formula $R_{10}$——$R_{11}$ wherein $R_{10}$ is a lower alkylene group or a lower alkenylene group, and $R_{11}$ is a carboxyl group, a carboxylic ester group or a heterocyclic group], and $R_7$ is a single bond or a group of the formula $-CONH\underset{R_{12}}{\underset{|}{C}}H-$ [wherein $R_{12}$ is a lower alkyl group, an optionally-substituted phenyl group or an optionally-substituted heterocyclic group].

Specific examples of acyl groups of this type are 2-(2-aminothiazol-4-yl)-(Z)-substituted-oxyiminoacetyl groups of the formula:

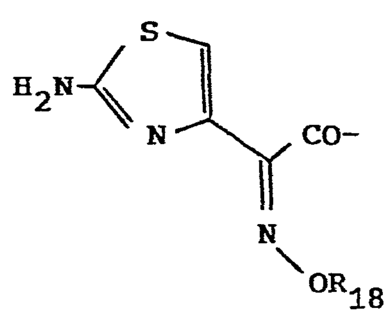

wherein $R_{18}$ is a hydrogen atom or a straight-chain or branched-chain alkyl, alkenyl or alkynyl group which contains up to 12 carbon atoms and which may optionally be substituted. Preferred examples thereof are those wherein $R_{18}$ is methyl.

Further specific examples of the acyl groups of this type are 2-(2-aminothiazol-4-yl)-(Z)-2-substituted-oxyiminoacetyl groups of the formula:

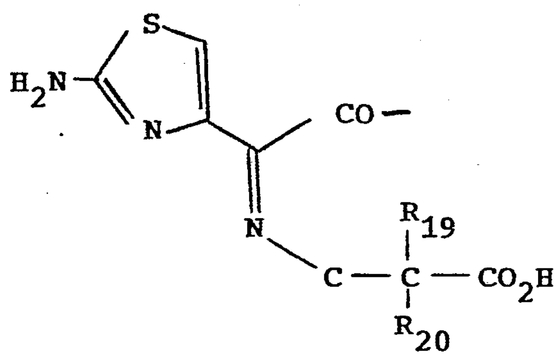

wherein $R_{19}$ and $R_{20}$ are the same or different and each is a $C_1 - C_4$ alkyl group or $R_{19}$ and $R_{20}$ are combined together with the carbon atom to which they are attached to form a $C_3 - C_7$ cycloalkylidene group. Preferred examples thereof are those wherein $R_{19}$ and $R_{20}$ are both methyl.

(4) Acyl groups of the formula:

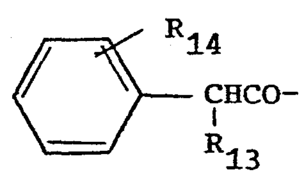

wherein $R_{13}$ is a hydroxyl group, a hydroxysulphonyloxy group, a carboxyl group, an optionally-substituted sulphamoyl group, a sulphonic acid group, a benzyloxycarbonyl group, a formyloxy group, an azido group, or a halogen atom, and $R_{14}$ is a hydrogen atom, a lower alkyl group, a halogen atom, an azido group, a nitro group or a hydroxyl group.

The term "lower" herein means a group preferably

containing from 1 to 4 carbon atoms.

Examples of the amino-protecting group $R_1$ include aromatic acyl groups such as phthaloyl, p-nitrobenzoyl, p-tert-butylbenzoyl and p-tert-butylbenzene sulphonyl groups; aliphatic acyl groups such as formyl, acetyl, propionyl, monochloroacetyl, dichloroacetyl and trichloroacetyl groups; esterified carboxyl groups such as methoxycarbonyl, ethoxycarbonyl, t-butoxycarbonyl, isopropoxycarbonyl, benzyloxycarbonyl, p-nitrobenzyloxycarbonyl and p-methoxybenzyloxycarbonyl groups; trityl groups; and 2-nitrophenylthio groups.

Examples of the alkyl group in the alkyloxy group X include lower alkyl such as methyl, ethyl and butyl groups. Examples of the aralkyl group in the aralkyloxy group include benzyl, phenetyl and p-nitrobenzyl groups. Examples of the aryl group in the aryloxy group X include phenyl and p-nitrophenyl groups.

The derivatives wherein $R_1$ is an amino-protecting group, may be produced by, for example, the following process.

(1) A compound of the general formula:

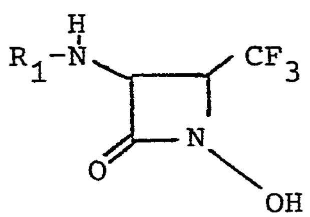

(II)

is reacted with titanium trichloride in water or in a mixed solvent of water and alcohol to obtain an azetidinone derivative of the general formula:

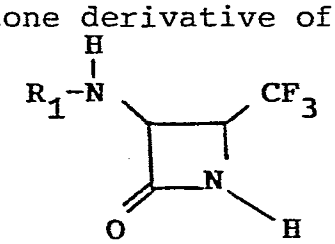

(III)

The thus obtained derivative is reacted with sulphuric anhydride or a reactive derivative thereof to obtain an azetidinone derivative of the general formula:

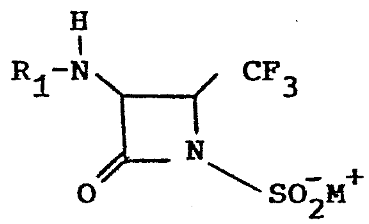

(IV)

wherein $M^+$ is a cation.

(2) A compound of the general ;formula:

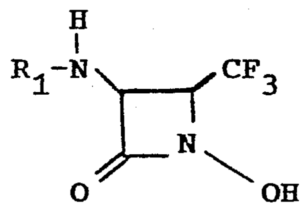

(V)

is reacted with sulphuric anhydride or a reactive derivative thereof to obtain an azetidinone derivative of the general formula:

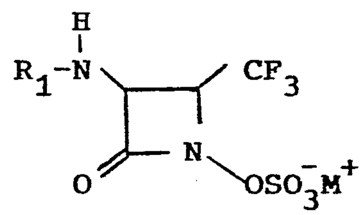

(VI)

wherein $M^+$ is a cation.

A compound of the general formula:

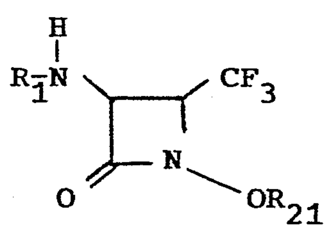

(VII)

may be produced by subjecting a compound of the general formula:

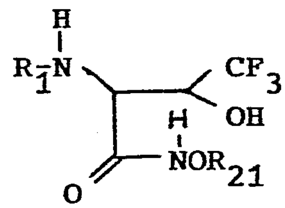

(VIII)

[which is obtained by, for example, protecting the amino group of 2-amino-3-hydroxy-4,4,4-trifluorobutanoic acid (see Journal of American Chemical Society, 80, 187 - 192 (1958)) and reacting this with an O-alkyl- or O-aralkylhydroxylamine using a carbodiimide as a condensing agent] to a dehydrocyclization reaction using triphenylphosphine and diethyl azodicarboxylate. In the above, $R_1$ is as defined above, and $R_{21}$ is an alkyl group, an aralkyl group or an aryl group.

Specific examples of the amino-protecting group used for protecting the free amino group include conventional amino-protecting groups, such as groups forming a urethane group with the amino group (e.g. t-butoxycarbonyl, cyclopentyloxycarbonyl, 2,2,2-trichloroethoxycarbonyl, benzyloxycarbonyl, p-nitrobenzyloxycarbonyl, diphenylmethoxycarbonyl, etc.), a trityl group, etc., among which t-butoxycarbonyl is particularly preferred. Condensation of a compound of the following formula:

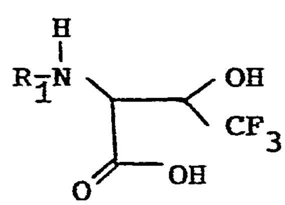

(IX)

wherein the amino group is protected, with an O-alkyl- or O-aralkylhydroxylamine may be effected in a mixed solvent such as water and tetrahydrofuran (THF) or dimethylformamine (DMF) and water, and the reaction temperature is usually -5 to 40°C, preferably 0 to 20°C. As the condensing agent,

a carbodiimide may be used; in particular, 1-ethyl-3-(3-dimethylamino)propyl)-carbodiimide is preferred. The hydroxylamine is preferably used, relative to the starting material, in an amount of from 1 to 2.0 molar, more preferably from 1 to 1.5 molar, and the carbodiimide is preferably used in an amount of from 1 to 2.0 molar, more preferably from 1 to 1.5 molar.

The dehydrocyclization reaction of the compound (VIII) may be effected in an inert etheric solvent such as tetrahydrofuran, diethyl ether, dioxane, etc., among which tetrahydrofuran is particularly preferred. The triphenylphosphine is preferably used, relative to the compound (VIII), in an amount of from 1 to 3 molar, more preferably from 1 to 1.5 molar, and the diethyl azodicarboxylate is preferably used in an amount of from 1 to 3 molar, more preferably from 1 to 1.5 molar. The reaction temperature usually is from -30°C to the refluxing temperature of the solvent, preferably from -10°C to 25°C. The reaction time is usually from 1 to 30 hours, preferably from 15 to 20 hours. The compound (II) may be produced by subjecting the compound (VII) wherein $R_{21}$ in the general formula is benzyl or p-nitrobenzyl to a hydrogenation reaction in a solvent such as ethyl acetate, dioxane, tetrahydrofuran, methanol, ethanol, etc., in the presence of a hydrogenating catalyst such as platinum metal or its oxides, 10% Pd/C, 30% Pd/$BaCO_3$, etc., preferably at 0 to 20°C for 5 minutes to 2 hours under a hydrogen pressure of from 1 to 10 atmospheres. The compound (III) may be produced by reducing the compound (II) in a mixed solvent of alcohol and water under neutral conditions. The reaction temperature is preferably 0 to 30°C, more preferably 0 to 10°C, and the reaction time is usually 1 to 2 hours.

The compounds (IV) and (VI) may be produced by reacting the compound (II) or (III) with, for example, sulphuric anhydride or a reactive derivative of sulphuric anhydride. Examples of the reactive derivatives of sulphuric anhydride are adducts such as sulphuric anhydride-pyridine, sulphuric anhydride-α-picoline, sulphuric anhydride-dimethylformamide, sulphuric anhydride-dioxane, chlorosulphonic acid-pyridine, chlorosulphonic acid-α-picoline, etc. The reaction is usually effected by adding about 1 to about 5 moles, preferably about 1 to about 2 moles, of sulphuric anhydride or a reaction derivative of sulphuric anhydride per mole of the compound (II) or (III). The reaction temperature is usually -10 to 80°C, preferably 0 to 30°C. As the reaction solvent, ethers (such as dioxane, THF, diethyl ether, etc.), esters (such as ethyl acetate, ethyl formate, etc.), halogenated hydrocarbons (such as chloroform, methylene dichloride, etc.), hydrocarbons (such as benzene, n-hexane, etc.), dimethylformamide, dimethylacetamide, etc., are preferably employed, either singly or in combination. After completion of the reaction, the reaction mixture may be treated by known purifying and separating mens, such as solvent extraction, recrystallization, chromatography, etc., to obtain a product of a desired purity.

The cationic moieties of the sulphonic acid salts of the azetidinone derivatives (IV) and (VI) may be derived from either an organic or an inorganic base. Examples of such cationic moieties include the following ions: ammonium; substituted ammonium such as alkylammonium (e.g. tetra-n-butylammonium), etc.; alkali metal such as lithium, sodium, potassium, etc.; alkaline earth metal such as calcium, magnesium, etc.; pyridinium; and dicyclohexylammonium.

The compounds (X) wherein $R_1$ is hydrogen:

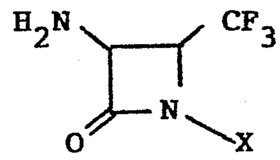

(X)

wherein X is as defined above may be obtained by removing the amino-protecting group from compounds of the general formula (I) wherein $R_1$ is an amino-protecting group. The method used in this respect may be one conventionally employed in peptide synthetic chemistry, for example, the method described in Tanpakushitsu Kagaku 1, Amino-san Pepuchido (Protein Chemistry 1, Amino Acid - Peptide) ed. by Akabori, Kaneko and Narita, Kyoritsu Shuppan, 1969.

In a particular, the compounds (XI) and (XII) wherein X is $-SO_3H$ or $-OSO_3H$:

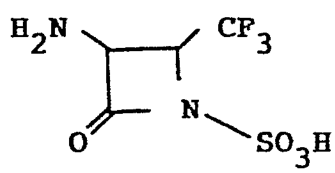

(XI)

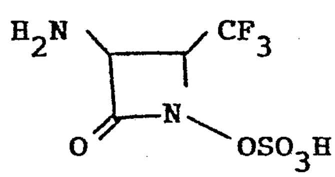

(XII)

are important as nuclei for novel, highly active β-lactam antibiotics.

For the production of compounds wherein $R_1$ is an acyl group, there are preferably used the following two processes, namely a process which comprises synthesizing the compound (X) wherein $R_1$ is H and thereafter acylating it, and a process which comprises synthesizing the compound (IX) or

(VII) wherein $R_1$ is acyl and thereafter cyclizing it. Examples of the acylating agent include acid halides, in particular, acid chlorides and bromides. Such acylating agents may be prepared by reacting an acid or a salt thereof with a halogenating agent such as phosphorus pentachloride, thionyl chloride, oxalyl chloride, etc. The acylation using an acid halide may be effected in an aqueous or non-aqueous solvent at a preferred temperature of from -50 to 50°C, more preferably from -30 to 20°C, in the presence of an acid binding agent. Examples of suitable reaction solvents include aqueous ketones such as aqueous acetone, esters such as ethyl acetate, halogenated hydrocarbons such as methylene chloride, amides such as DMF, nitriles such as acetonitrile, ethers such as THF, and mixtures of two or more thereof. Examples of suitable acid binding agents include tertiary amines (e.g. triethylamine, dimethylaniline, diisopropylethylamine, etc.), inorganic salts (e.g. sodium bicarbonate, calcium carbonate, etc.) and oxylanes such as lower 1,2-alkylene oxides which combine with the hydrogen halide liberated during the acylation reaction (e.g. ethylene oxide and propylene oxide). In addition, a carboxylic acid per se may be used as the acylating agent. The acylatation using a carboxylic acid may be effected in the presence of a condensing agent, for example, a carbodiimide such as N,N'-dicyclohexylcarbondiimide, 1-ethyl-3-(3-(dimethylamino)propyl)carbodiimide, etc., a carbonyl compound such as carbonyldiimidazole, or an isoxazolium salt such as N-ethyl-5-phenylisoxazolium perchlorate.

The acylation may also be effected by reacting a reactive derivative of other carbonyl groups. Specific examples thereof include reactive derivatives with an acid anhydride, a mixed acid anhydride,

an active ester, an acid azide, Vilsmeier reagent, etc. Examples of the mixed acid anhydride include mixed acid anhydrides with carbonic acid monoalkyl esters such as monoethyl carbonate, monoisobutyl carbonate, etc., mixed acid anhydrides with optionally halogenated lower alkanoic acids such as pivalic acid, trichloroacetic acid, etc., mixed acid anhydrides with alkylsulphonic acids such as methanesulphonic acid, ethanesulphonic acid, etc., and the like. Examples of the active ester include substituted phenyl esters, benzotriazole esters, succinimide esters, etc. Examples of the reactive derivative with a reagent include reactive derivatives with Vilsmeier reagent obtained by reacting an acid amide such as N,N'-dimethylformamide, N,N'-dimethylacetamide, etc. with an acyl halogenating agent such as phosgene, thionyl chloride, phosphorus trichloride, phosphorus oxychloride, trichloromethyl chloroformate, etc. Examples of suitable solvents employed in the reactions with these reactive derivatives include ethers such as diethyl ether, THF, etc., halogenated hydrocarbons such as methylene chloride, chloroform, etc., aromatic hydrocarbons such as benzene, toluene, etc., ketones such as acetone, etc., and mixtures of two or more thereof. Further, the reaction may also be effected in the presence of a base. Examples of the base include tertiary amines such as triethylamine, dimethylaniline, diisopropylethylamine, etc. While the reaction temperature and the reaction time are not particularly restricted, it is preferred to effect the reaction at -50 to 30°C, and at that temperature, the reaction is usually completed in several hours. Furthermore, the aforesaid acylated reaction may also be effected in the presence of a catalyst such as 4-dimethylaminopyridine.

Of the compounds of the general formula (I)

according to the present invention, those derivatives wherein $R_1$ is an acyl group have been found to have a wide antibacterial spectrum against Gram negative bacteria, including Pseudomonas aeruginosa, and Gram positive bacteria, and thus can be used as antibiotics.

The compounds of the present invnetion also include their salt forms, as described above. Cations for forming these salts are those know in this art, and examples thereof include alkali metal ions such as lithium, sodium, potassium, etc. alkaline earth metal ions such as magnesium, calcium, etc., and optionally substituted ammonium ions such as ammonium, tetra-n-butylammonium, pyridinium, dicyclohexylammonium, etc.

Furthermore the derivatives of the present invention may exist in the form of optical isomers, and in such a case, the respective isomers and also their mixtures are within the scope of the present invention.

That the compounds of the present invention have the above-described chemical structures has been confirmed by elementary analysis, infrared absorption spectrum, nuclear magnetic resonance spectrum, etc.

In the production of the derivatives of the present invention, the method of protecting the amino group and the method of removing the protecting group may be one of the methods conventionally employed in peptide synthetic chemistry, for example the method described in Tanpakushitsu Kagaku 1, Amino-san Pepuchido (Protein Chemistry 1, Amino Acid . Peptide) ed. Akabori, Kaneko, and Narita, Kyoritsu Shuppan, 1969.

The present invention is illustrated by the following Examples.

Example 1

Production of 3-t-butoxycarboxamido-1-benzyloxy-4-trifluoromethyl-2-azetidinone

(a) Production of 2-t-butoxycarboxamido-3-hydroxy-4,4,4-trifluorobutanoic acid

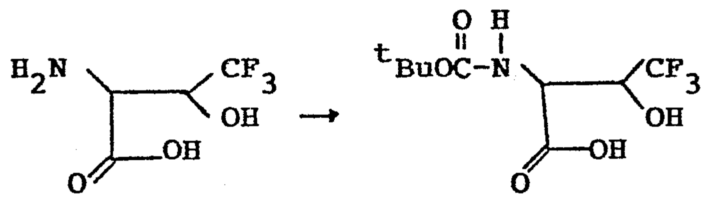

2-Amino-3-hydroxy-4,4,4-trifluorobutanoic acid (2 g, 11.6 mM) was dissolved in a mixed solvent of 10 ml of THF and 20 ml of water. Triethylamine (1.62 ml, 11.6 mM) and di-t-butyl dicarbonate (3.2 g, 14.7 mM) were added thereto, and the mixture was stirred at room temperature for 15 hours.

After completion of the reaction, the THF was distilled off under reduced pressure, and the pH was adjusted to 2.5 with 1N hydrochloric acid, with ice cooling. The resulting aqueous solution was extracted three times with 30 ml of ethyl acetate, and the organic layers were combined, washed with 40 ml of water, and then washed with 30 ml of saturated brine. The solution was dried over anhydrous sodium sulphate, the solvent was evaporated off under reduced pressure, and the residue was re-precipitated with n-hexane to obtain the crude title compound (2.2 g, yield 70%).

$IR_{\nu max}^{KBr}(cm^{-1})$ : 3300, 1730, 1645, 1520, 1258, 1160, 1140.

NMR ($DMSO-d_6$,ppm) : 1.40 (S, 9H), 4.20 (m, 2H).

(b) Production of O-benzyl-2-t-butoxycarboxamido-3-hydroxy-4,4,4-trifluorobutanohydroxamate

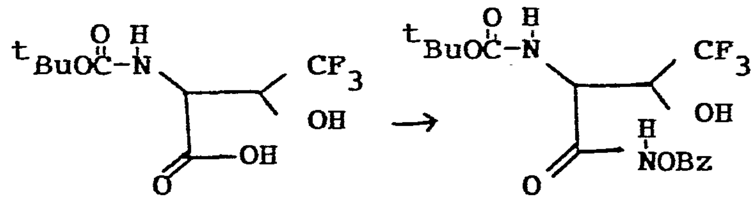

2-t-Butoxycarboxamido-3-hydroxy-4,4,4-trifluorobutanoic acid (1.90 g, 6.96 mM) was dissolved in a mixed solvent of 15 ml of THF and 15 ml of water, and then O-benzylhydroxylamine hydrochloride (1.33 g, 8.36 mM) was added. The pH of this solution was adjusted to 4.0 - 5.0 with a saturated aqueous sodium bicarbonate solution, and then an aqueous solution of 1-ethyl-3-(3-(dimethylamino)propyl)carbodiimide hydrochloride (water-soluble carbodiimide) (1.46 g, 7.64 mM) was added dropwise thereto, with ice cooling. The pH of the solution during the addition, and the reaction, was adjusted to 4.5 - 5.0 with 1N hydrochloric acid. The reaction was effected with ice cooling for 20 minutes and then at room temperature for 30 minutes. Then, the THF was evaporated off under reduced pressure, and the residue was extracted three times with 40 ml of ethyl acetate. All of the organic layers were combined, washed with a 0.5M aqueous citric acid solution, then with 5% aqueous sodium bicarbonate solution and further with saturated brine. The solution was dried over anhydrous sodium sulphate, and the ethyl acetate was evaporated off to obtain 1.88 g (yield 70%) of the crude title compound.

$IR_{\nu max}^{KBr}(cm^{-1})$ : 3400, 3300, 3200, 1680, 1660, 1520, 1300, 1260, 1160, 1120, 740, 960.

NMR($DMSO-d_6$,ppm) : 1.41 (S, 9H), 4.20 (m, 2H), 4.82 (S, 2H), 6.40 (broad S, 1H), 7.35 (S, 5H).

(c) Production of 3-t-butoxycarboxamido-1-benzyloxy-4-trifluoro-methyl-2-azetidinone

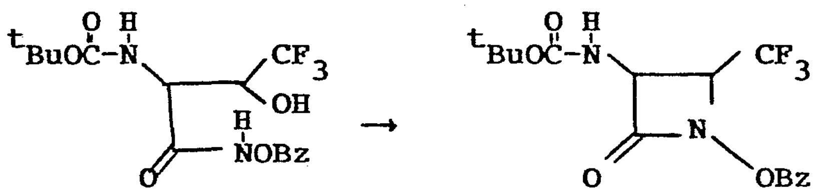

O-benzyl-2-t-butoxycarboxamido-3-hydroxy-4,4,4-trifluorobutanohydroxamate (0.70 g, 1.85 mM) and triphenylphosphine (0.58 g, 2.21 mM) were dissolved in 15 ml of dry THF in a nitrogen stream. A solution of diethyl azodicarboxylate (0.35 g, 2.03 mM) dissolved in 10 ml of dry THF was added dropwise thereto with ice cooling over 30 minutes. After 30 minutes, this was allowed to warm to room temperature, and stirred at the same temperature for 20 hours. The solvent was evaporated off, and the residue was then subjected to silica gel chromatography. Elution with a mixture of ethyl acetate and n-hexane (1:3) gave 0.21 g (yield 30%) of 3-t-butoxycarboxamido-1-benzyloxy-4-trifluoromethyl-2-azetidinone as crystals.

$IR^{KBr}_{\nu max}(cm^{-1})$ : 3320, 1805, 1698, 1523, 1285, 1160, 1140.

NMR($DMSO-d_6$,ppm) : 1.40 (s, 9H), 4.80 (m, 1H), 4.94 (S, 2H), 5.15 (m, 1H), 7.40 (S, 5H), 8.00 (d, 1H).

Example 2

Production of 3-t-butoxycarboxamido-1-hydrody-4-trifluoromethyl-2-azetidinone

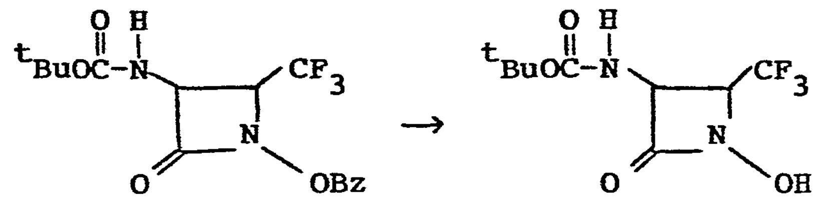

3-t-Butoxycarboxamido-1-benzyloxy-4-trifluoromethyl-2-azetidinone (70 mg, 0.19 mM) was dissolved in 10 ml of methanol, and, after adding 10% Pd/C (20 mg), the mixture was stirred at normal temperature and normal pressure in a hydrogen stream for 45 minutes. The catalyst was filtered off, and the solvent was evaporated off under reduced pressure to obtain 38 mg (yield 72%) of the title compound.

$IR^{KBr}_{\nu max}(cm^{-1})$ : 3310, 1780, 1752, 1690, 1517, 1290, 1160, 940, 870.

NMR($CDCl_3$, ppm) : 1.42 (S,9H), 4.33 (m, 1H), 5.11 (dd, 1H).

The obtained compound may be converted to the free form by treating with trifluoroacetic acid.

**Example 3**

**Production of 3-t-butoxycarboxamido-4-trifluoromethyl-2-azetidinone**

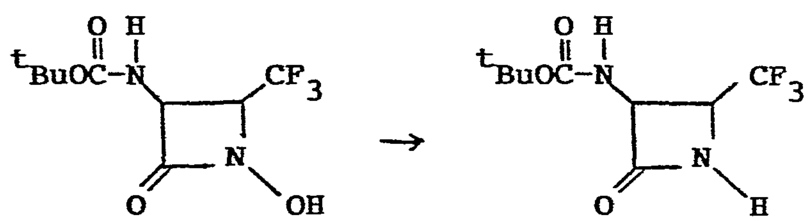

3-t-Butoxycarboxamido-1-hydroxy-4-trifluoromethyl-2-azetidinone (140 mg, 0.52 mM) was dissolved in a mixed solvent of 8ml of methanol and 12 ml of water, and ice cooled in a nitrogen stream. Sodium acetate (0.6g) was added and dissolved, and, while maintaining the pH at 7.5 - 6.0, 20% aqueous titanium trichloride solution (1.8 ml) was added

dropwise with ice cooling. The reaction was effected at 0 - 5°C for 30 minutes and then at room temperature for 30 minutes. After completion of the reaction, extraction with 20 ml of ethyl acetate was conducted three times. The organic layers were then combined, and washed with 30 ml of a 0.5M aqueous citric acid solution, then with 5% aqueous $NaHCO_3$ solution and further with saturated brine. The solution was dried over anhydrous sodium sulphate, and the ethyl acetate was evaporated off to obtain 110 mg (yield 83%) of the crude title compound.

$IR_{\nu max}^{KBr}(cm^{-1})$ : 3300, 1780, 1690, 1520, 1280, 1150, 1070, 850.

NMR(DMSO-$d_6$, ppm) : 1.39 (s, 9H), 4.38 (m, 1H), 5.20 (dd, 1H), 7.92 (d, 1H), 8.87 (s, 1H).

**Example 4**

**Production of 3-t-butoxycarboxamido-4-trifluoromethyl-2-azetidinone-1-sulfonic acid tetrabutylammonium salt**

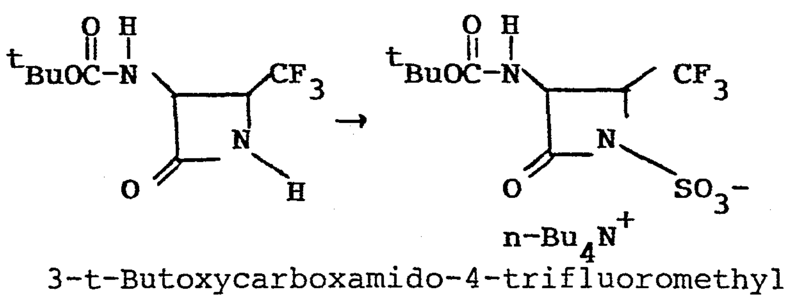

3-t-Butoxycarboxamido-4-trifluoromethyl-2-azetidinone (100 mg, 0.39 mM) was dissolved in 20 ml of dry methylene chloride in a nitrogen stream. An α-picoline-sulphuric anhydride complex (0.30g) was added thereto, and the mixture was stirred at room temperature for 3 days. The reaction mixture was added to 20 ml of 0.5M aqueous $KH_2PO_4$ solution, and extracted into the aqueous layer. The methylene chloride layer was further extracted with 20 ml of 0.5M aqueous $KH_2PO_4$ solution. The aqueous layers

were then combined, and, after adding 0.13 g of tetrabutylammonium hydrogensulphate, extracted with 40 ml of methylene chloride. The aqueous layer was further extracted with 40 ml of methylene chloride. The organic layers were then combined, dried over anhydrous sodium sulphate, and the methylene chloride was evaporated off to obtain 130 mg (yield 60%) of the crude title compound as an oil.

$IR_{\nu max}^{neat}(cm^{-1})$ : 3300, 2950, 2850, 1790, 1720, 1280, 1250, 1160, 1140, 1045.

NMR($CDCl_3$, ppm) : 1.00 (m, 12H), 1.45 (m, 17H) 1.65 (m, 8H), 3.25 (m, 8H) 4.55 (m, 1H), 5.08 (d, 1H), 5.35 (dd, 1H).

**Example 5**

**Production of 3-amino-4-trifluoromethyl-2-azetidinone-1-sulphonic acid**

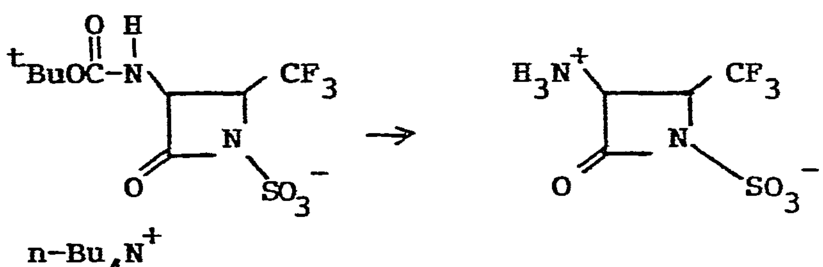

3-t-Butoxycarboxamido-4-trifluoromethyl-2-azetidinone-1-sulphonic acid tetrabutylammonium salt (100 mg, 0.18 mM) was dissolved in 3 ml of dry methylene chloride in a nitrogen stream. With ice cooling, 0.5 ml of anisole and 1 ml of trifluoroacetic acid were added thereto, and the mixture was stirred at 0 - 5°C for 40 minutes. The methylene chloride and the trifluoroacetic acid were evaporated off under reduced pressure, and diethyl ether was added to precipitate the product, namely 23 mg (yield 65%) of a zwitter-ion of the title compound.

$IR_{\lambda max}^{KBr}(cm^{-1})$ : 3400, 3000, 1800, 1320, 1300, 1270, 1200, 1150.

NMR($D_2O$, ppm) : 5.50 (m, 1H), 5.15 (d, 1H).

**Example 6**

**Production of 3-t-butoxycarboxamido-4-trifluoromethyl-2-azetidinone-1-oxysulphonic acid tetrabutylammonium salt**

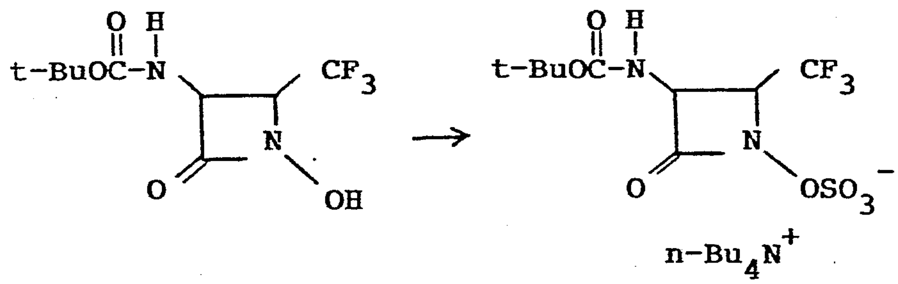

3-t-Butoxycarboxamido-1-hydroxy-4-trifluoromethyl-2-azetidinone (100 mg, 0.37 mM) was dissolved in 20 ml of dry methylene chloride in a nitrogen stream. An α-picoline-sulphuric anhydride complex (0.30g) was added thereto, and the mixture was stirred at room temperature for a day. The reaction mixture was added to 20 ml of 0.5M aqueous $KH_2PO_4$ solution. The aqueous layers were combined, and, after adding 0.13 g of tetrabutylammonium hydrogensulphate, extracted with 40 ml of methylene chloride. The aqueous layer was further extracted with 40 ml of methylene chloride. The organic layers were combined, dried over anhydrous sodium sulphate, and the methylene chloride was evaporated off to obtain 157 mg (yield 72%) of the crude title compound.

$IR_{\lambda max}^{neat}(cm^{-1})$ : 3300, 2950, 2850, 1800, 1710, 1280, 1150, 1050.

NMR($CDCl_3$, ppm) : 1.00 (m, 12H), 1.45 (m, 17H), 1.65 (m, 8H), 3.25 (m, 8H), 5.00 (m, 2H), 5.30 (dd, 1H).

Example 7

Production of 3-[D(-)-α-(4-ethyl-2,3-dioxo-1-piperazinecarboxamido)-α-phenylacetamido]-4-trifluoromethyl-2-azetidinone-1-sulphonic acid tetrabutylammonium salt

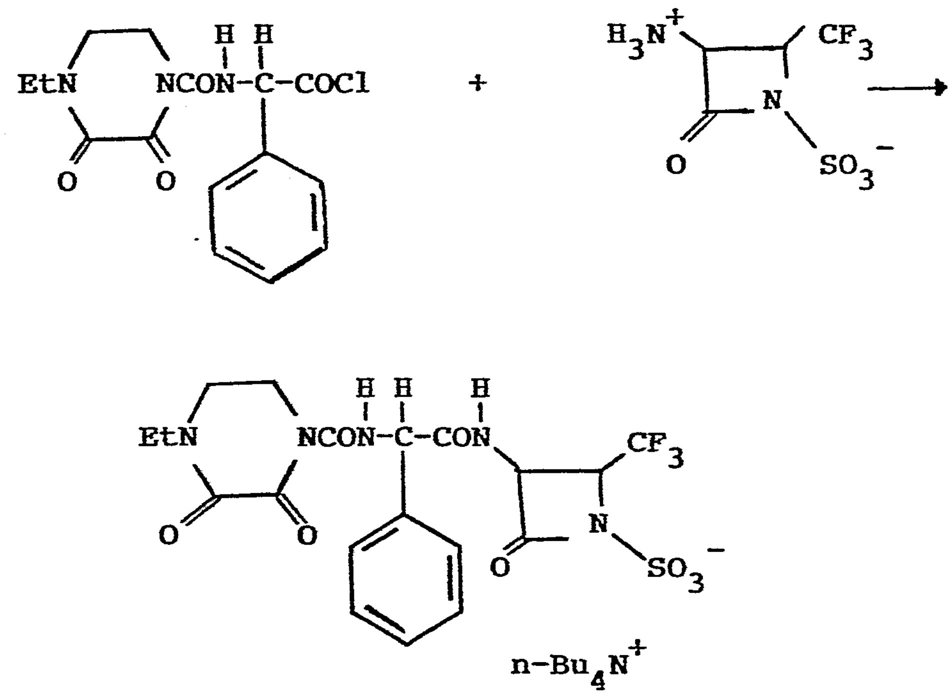

In a nitrogen stream, D(-)-α-(4-ethyl-2,3-dioxo-1-piperazinecarboxamido)phenylacetic acid (95 mg, 0.28 mM) was dissolved in dry methylene chloride, 76 mg of oxalyl chloride and one drop of dimethylformamide were added with ice cooling, and the mixture was stirred at room temperature for an hour. The solvent was evaporated off under reduced pressure, and 4 ml of dry tetrahydrofuran were added to obtain an acid chloride solution. Separately, in a nitrogen stream, 3-amino-4-trifluoromethyl-2-azetidinone-1-sulphonic acid (30 mg, 0.15 mM) and 4-dimethylaminopyridine (5 mg, 0.04 mM) were dissolved in 1 ml of dry dimethylformamide, and diisopropylethylamine (65 mg, 0.5 mM) was added thereto to obtain a uniform solution.

This solution was cooled to -10° to -5°C, and the solution of the acid chloride in tetrahydrofuran obtained above was added dropwise thereto. The reaction was effected at -10° to -5°C for 30 minutes and further at room temperature for an hour. After completion of the reaction, the reaction mixture was poured into 10 ml of ice water, the pH was adjusted to 6.5 - 7.0 with aqueous $NaHCO_3$ solution, the tetrahydrofuran was evaporated off under reduced pressure, and the aqueous layer was washed with 10 ml of ethyl acetate. The pH of the aqueous layer was adjusted to 2.5 with 5% hydrochloric acid, and was then washed twice with 10 ml of ethyl acetate. The pH of the aqueous layer was adjusted to 6.5 - 7.0, then 50 mg of tetrabutylammonium hydrogensulphate were added, the extraction with 15 ml of methylene chloride was conducted three times. The methylene chloride layers were combined, washed with 10 ml of water, dried over anhydrous sodium sulphate, and the solvent was evaporated off. Diethyl ether was added to the residue to precipitate the product, namely 63 mg (yield 55%) of the crude title compound as an oil.

$IR_{\lambda max}^{neat}(cm^{-1})$ : 3300, 2950, 2850, 1790, 1670, 1250, 1150, 1035.

$NMR(CDCl_3\ ppm)$ : 1.00 (m, 12H), 1.20 (t, 3H), 1.45 (m, 8H), 1.65 (m, 8H), 3.25 (m, 8H), 3.55 (q, 2H), 3.60 - 4.20 (m, 4H), 4.65 (m, 1H), 5.57 (d, 1H), 5.70 (dd, 1H), 7.30 - 7.40 (m, 5H), 7.59 (d, 1H).

**Example 8**

**Production of 3-[2-(2-aminothiazol-4-yl)-(Z)-2-methoximinoacetamino]-4-trifluoromethyl-2-azetidinone-1-sulphonic acid**

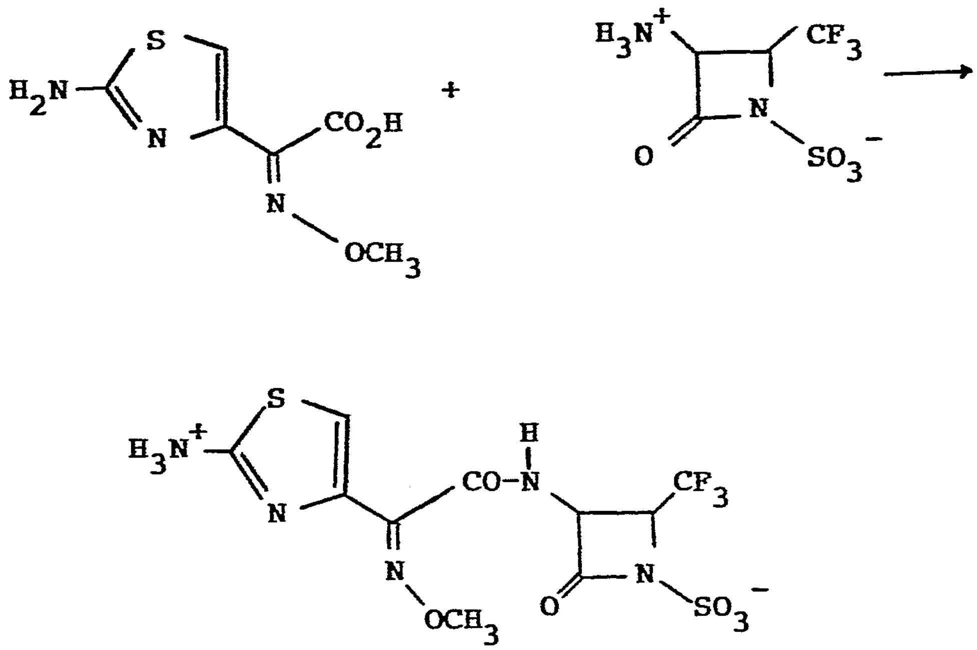

In a nitrogen stream, 2-(2-aminothiazol-4-yl)-(Z)-2- methoximinoacetic acid (136 mg, 0.68 mM) was suspended in 4 ml of dry methylene chloride, 210 mg of phosphorus oxychloride and 59 mg of dimethylformamide were added, and reaction was effected at -15° to -10°C for 2 hours. To this reaction mixture was added dropwise a mixed solution of 3-amino-4-trifluoromethyl-2-azetidinone-1-sulphonic acid (102 mg, 0.50 mM), 6 ml of dry methylene chloride and 240 µl of diisopropylethylamine. Reaction was effected at -10°C for an hour, and then at room temperature for 2 hours. After completion of the reaction, the reaction mixture was dissolved in 20 ml of ice water, the pH was adjusted to 7.0 with 20 ml of ethyl acetate, and the aqueous layer was concentrated to 5 ml under reduced pressure. The aqueous layer was then purified by column chromatography using "Amberlite XAD-2" (a highly porous polymer produced by Rohm & Haas Co.) and a mixture of tetrahydrofuran and water (1:10) as an eluting agent, and by freeze drying to obtain

29 mg (yield 15%) of the title compound.

$IR_{\lambda max}^{KBr}(cm^{-1})$ : 1795, 1650, 1280, 1150, 1040.

NMR($DMSO-d_6$,ppm) : 3.95 (s, 3H), 4.75 (m, 1H), 5.40 (dd, 1H) 6.95 (s, 1H).

We have confirmed that the compounds obtained by the aforegoing Examples 7 and 8 show a wide antibacterial spectrum against Gram negative bacteria, including Pseudomonas aeruginosa, and Gram positive bacteria.

CLAIMS

1. An azetidinone derivative of the general formula:

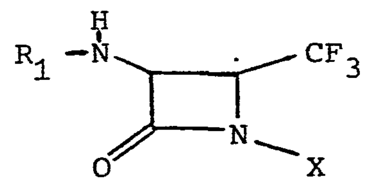

wherein $R_1$ is an acyl group, a hydrogen atom or an amino-protecting group, and X is a hydrogen atom, an alkyloxy group, an aralkyloxy group, an aryloxy group, the group -OH, the group $-OSO_3^-M^+$ wherein $M^+$ is a cation, or the group $-SO_3^-M^+$ wherein $M^+$ is a cation.

2. A derivative according to claim 1, wherein X is the group $-OSO_3H$ or the group $-SO_3H$ in a salt form.

3. A derivative according to claim 1 or 2, wherein $R_1$ is an acyl group of the formula:

$$R_2CO-$$

wherein $R_2$ is a lower alkyl group, an optionally-substituted phenyl group, a heterocyclic group, or an optionally-substituted benzoyl group.

4. A devivative according to claim 1 or 2, wherein $R_1$ is an acyl group of the formula:

$$R_3NH\underset{\underset{R_5}{|}}{C}HCO-$$

wherein $R_3$ is a hydrogen atom, an optionally-substituted amino acid residue, an amino-protecting group, a group of the formula $R_4-(CH_2)_{n'}-CO-$ [wherein $R_4$ is an optionally-substituted heterocyclic group, an optionally-substituted phenyl group, an optionally-substituted lower alkyl group, an optionally-substituted phenylthio group, or an optionally-substituted lower alkylthio group, n' is 0 or an integer of 1 to 4, and $-(CH_2)_{n'}-$ may optionally contain one or more substituents], a group of the

formula 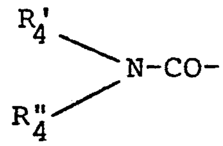

[wherein $R_4'$ and $R_4''$ are the same or different and each is a hydrogen atom, a lower alkyl group, a lower alkylcarbamoyl group, an optionally-substituted phenylcarbamoyl group, or an optionally-substituted sulphonic acid group, or $R_4'$ and $R_4''$ together are a cyclic hydrocarbon ring which may optionally contain one or more nitrogen atoms and/or one or more oxygen atoms and/or one or more other hetero atoms and whose carbon atom(s) and/or nitrogen atom(s) may optionally contain substituents], or a group of the formula $R_4''' -SO_2-$ [wherein $R_4'''$ is an optionally-substituted lower alkyl group]; and $R_5$ is a hydrogen atom, an optionally substituted lower alkyl group, an optionally-substitued phenyl group, an optionally-substituted heterocyclic group, a cycloalkenylene group, or a combination of a heterocyclic ring and carbonylamino group which heterocyclic ring and carbonylamino group may optionally be combined via an optionally-substituted alkylene group.

5. A derivative according to claim 1 or 2, wherein $R_2$ is an acyl group of the formula:

$$R_6-R_7-CO-$$

wherein $R_6$ is a group of the formula $R_8-\overset{|}{C}=N-X'-R_9$ [wherein X' is an oxygen or sulphur atom, $R_8$ is an optionally-substituted heterocyclic group or an optionally-substituted phenyl gorup, and $R_9$ is a hydrogen atom, an optionally-substituted phenyl group, an optionally-substituted lower acyl group, an optionally-substituted lower alkyl group, or a group of the formula $R_{10}$—— $R_{11}$ wherein $R_{10}$ is a lower alkylene group or a lower alkenylene group, and $R_{11}$ is a carboxyl group, a carboxylic ester group or a heterocyclic group]; and $R_7$ is a single

bond or a group of the formula 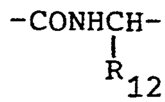

[wherein $R_{12}$ is a lower alkyl group, an optionally-substituted phenyl group or an optionally-substituted heterocyclic group].

6. A derivative according to claim 1 or 2, wherein $R_2$ is an acyl group of the formula:

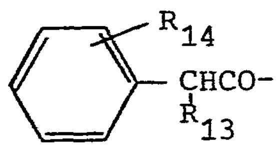

wherein $R_{13}$ is a hydroxyl group, a hydroxysulphonyloxy group, a carboxyl group, an optionally-substituted suphamoyl group, a sulphonic acid group, a benzyloxycarbonyl group, a formyloxy group, an azido group, or a halogen atom, and $R_{14}$ is a hydrogen atom, a lower alkyl group, a halogen atom, an azido group, a nitro group or a hydroxyl group.

7. 3-[D(-)-α-(4-ethyl-2,3-dioxo-1-piperazinecarboxamido)-α-phenyl-acetamido]-4-trifluoromethyl-2-azetidinone-1-sulphonic acid, or a salt thereof.

8. 3-[2-(2-aminothiazol-4-yl)-(Z)-2-methoxyiminoacetamido]-4-trifluoromethyl-2-azetidinone-1-sulphonic acid, or a salt thereof.

0117053

European Patent Office

# EUROPEAN SEARCH REPORT

Application number

EP 84300256.9

| Category | DOCUMENTS CONSIDERED TO BE RELEVANT<br>Citation of document with indication, where appropriate, of relevant passages | Relevant to claim | CLASSIFICATION OF THE APPLICATION (Int. Cl. 3) |
|---|---|---|---|
| P,Y | EP - A1 - 0 085 291 (SQIBB)<br>* Claim 1; line 9 *<br>-- | 1,3-6 | C 07 D 205/08<br>C 07 D 403/12<br>C 07 D 417/12<br>//A 61 K 31/395 |
| P,Y | EP - A1 - 0 083 039 (TAKEDA)<br>* Abstract; claims *<br>-- | 1-6 | |
| Y | EP - A1 - 0 051 381 (SQIBB)<br>* Claims *<br>-- | 1-6 | |
| Y | EP - A1 - 0 070 024 (SQIBB)<br>* Page 1 *<br>-- | 1,2,5,8 | |
| P,Y | DE - A1 - 3 239 157 (ROUSSEL-UCLAF)<br>* Claims *<br>-- | 1,2,5,8 | TECHNICAL FIELDS SEARCHED (Int. Cl. 3) |
| A | US - A - 4 174 316 (CHRISTENSEN et al.)<br>* Claim *<br>-- | 1 | C 07 D 205/00<br>C 07 D 403/00<br>C 07 D 417/00 |
| A | EP - A1 - 0 060 119 (LILLY)<br>* Abstract *<br>---- | 1 | |

The present search report has been drawn up for all claims

| Place of search | Date of completion of the search | Examiner |
|---|---|---|
| VIENNA | 05-04-1984 | JANISCH |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503. 03.82